# EUROPEAN PATENT APPLICATION

(11) **EP 3 173 119 A1**
(43) Date of publication of application: **31.05.2017**
(21) Application number: 16200269.5
(22) Date of filing: 23.11.2016
(51) Int. Cl.: A61M 25/02

(54) **WEARABLE DEVICE PROTECTING PERIPHERAL AND CENTRAL VENOUS CATHETERS**

(30) Priority: 25.11.2015 IT UB20155901
(71) Applicant: Giacobbe, Mauro, Genova (IT); Morano, Giacomo Giuseppe, Roma (IT); Rettagliati, Massimo, Genova (IT); Perucci, Anna Gloria, Roma (IT)
(72) Inventor: Giacobbe, Mauro, Genova (IT); Morano, Giacomo Giuseppe, Roma (IT); Rettagliati, Massimo, Genova (IT); Perucci, Anna Gloria, Roma (IT)
(74) Representative: Karaghiosoff, Giorgio Alessandro

(57) **Abstract**

The present invention relates to a wearable device (1, 10) protecting peripheral or central venous catheters, comprising
- a tubular body (2) made of neoprene comprising two opposite open orifices (3, 4)
- two annular sealing lips (31, 41) provided in proximity of such open orifices (3, 4),
said annular sealing lips (31, 41) being made of smooth neoprene, such to generate a watertight seal when in contact with the user skin.

## Description

### TECHNICAL FIELD

The present invention relates to the field of wearable protective devices to be used on patients having central and peripheral venous catheters.

### PRIOR ART

Among the several catheters some are of the semi permanent type that remains in place for a long time in the patient, such as those known by the English acronym P.I.C.C. (Peripherally Inserted Central Catheter) and the like. The P.I.C.C. is a peripherally inserted central venous catheter that remains in place for medium term (few months); it is placed via the basilic, brachial and cephalic veins of upper limbs) and it comes out from the arm for few centimeters, by a portion that provides the insertion cone with the closure cap and two anchoring tabs.

Other kinds of catheters of this type can be different from P.I.C.C. but -generally- all have anchoring tabs or protrusions that have to be attached to the skin of the forearm, by means of sticking plasters, that should not be removed such to prevent the catheter, that comes out from the arm, from being displaced and consequently removed.

Thus it is particularly important for the patient - during washing operations - to prevent the catheter and particularly the plaster from getting wet (for example when taking a bath or a shower), increasing also the risk of infecting the device itself.

It is also important during such operations to prevent the protruding portion of the catheter from accidentally contacting other objects, such to avoid pain for the patient and possible damages to the device.

To this end, in prior art, sleeves are used to be worn on the arm, in a position overlapping the catheter, during all those operations that make this appropriate.

The sleeve is made of rubber, latex or the like and once it is worn a vacuum is generated inside it by means of a suction pump and a suction nozzle tightly fastened to the sleeve and communicating with the inner compartment delimited on one side by the arm and on the other side by the sleeve. The vacuum generates a compression seal against the limb, for example the arm and about the protruding portion of the catheter.

Thus it is possible to prevent the involved area from getting wet.

Although in principle it is a valid solution, however it has some drawbacks.

Firstly such sleeves, when vacuum is generated, press on the arm, often right on the catheter, causing a certain discomfort, if not pain, for the patient and however such condition is not ideal since it urges the device against the surrounding tissues.

Secondly as regards the vacuum generating operation an operator is required together with the patient, since his/her movements are hindered, due to obvious reasons (he/she can clearly use only one hand when the catheter is on one arm).

Still another drawback is the fact that both the sleeve and the suction equipment (pump or the like) have to be at his/her disposal.

Finally another drawback is the fact that for the sleeve pressed by external pressure against the device it is not rare to be torn accidentally or due to wear after a long use.

The document US 2008/045906 describes a tubular protection for catheters, both as a non-waterproof version and as a waterproof cover. This document generally discloses waterproof material for making the sleeve to be placed over the catheter region for protection. Moreover for the watertight seal, a waterproof strap with closing means of the Velcro® type or the like is described in order to obtain the watertight seal. Such means, even if able to generate a considerable tightening force, do not guarantee such an adhesion against the body to prevent fluids from passing and therefore such to guarantee a waterproof condition.

The document GB2411119 describes a sleeve made of a transparent plastic material, composed of a tubular element and such to run along the arm in order to sterilize the arm for a surgical operation. In this case this is an object of the "disposable" type, that has to be as waterproof but only in order not to let the sterilizing agent leaking to the outside. The function and aim therefore are in contradiction with those of the device according to the present invention.

The document WO97/28765 discloses a device to be fastened around the leg of an infant inside which a wire passes serving for monitoring possible apneas. The device is made in such a manner that the wire does not allow the child to be strangled with the wire when sleeping or during movements and its aim is not to protect a part of the body by a watertight seal. The device is made of neoprene, however generally neoprene has not sealing functions when used for clothes or similar products.

The document US2007/144547 describes a kind of "waterproof vest" with a breathing corrugated tube that allows a person subjected to tracheotomy intervention to perform sports activities such as swimming or snorkeling. Also in this case the aim of the invention is not the watertight protection of an anatomical part of the body.

### OBJECTS AND SUMMARY OF THE INVENTION

The object of the present invention is to overcome prior art drawbacks.

Particularly the object of the present invention is to provide a protective device that does not cause discomfort or pain for the patient when in use and that meets conditions of use of the catheter device, so called P.I.C.C. or the like.

Another object of the invention is to provide a protective device simple to be used and that, if necessary, can be worn by the patient himself/herself, without the help of dedicated operators and/or without specific equipment.

Still another object is to provide such a protective device that is less subjected to possible damages deriving from the use.

These and other objects of the present invention are achieved by a protective device according to the first annexed claim.

The idea at the base of the invention is to provide a protective device wearable by patients having a venous catheter, comprising a tubular body made of neoprene comprising two opposite open orifices, two annular sealing lips provided in proximity of such open orifices, said annular sealing lips being made of smooth neoprene, such to generate a watertight seal when in contact with the user skin.

Such solution allows inflation operations to be avoided and it makes the sleeve easily usable.

For being used it is sufficient for the patient to wear the sleeve while paying attention in placing it overlapping the region to be protected, where the catheter comes out from the arm, by elastically deforming the annular lips, that due to the inherent elasticity of the material, generate a watertight seal against the arm skin.

According to a further advantageous characteristic, in combination or as an alternative to the other above characteristics, the tubular body, in the non worn condition, has an inner surface and an outer surface and wherein each annular sealing lip is provided on said outer surface of the body at the opposite ends thereof, such that, in the worn condition, said opposite ends are folded to place the sealing lip in contact with the user skin.

According to another advantageous characteristic in combination or as an alternative to the other above characteristics, the inner surface of the body is made of lined neoprene such to facilitate wearing operations.

According to another advantageous characteristic in combination or as an alternative to the other above characteristics, the sealing lips are as one piece with the body, such to make the production cheaper.

According to another advantageous characteristic in combination or as an alternative to the other above characteristics, the sealing lips are applied to the body, such to make the production more versatile.

According to another advantageous characteristic in combination or as an alternative to the other above characteristics, it comprises two annular tubular extensions, connected to the body, and arranged at the end portions thereof provided with orifices, such to facilitate the grasping action when wearing it.

According to another advantageous characteristic in combination or as an alternative to the other above characteristics, the annular tubular extensions are as one piece with the body, which further comprises two inner sleeves on which said sealing lips are provided, such that the force necessary to wear the device is applied directly to portions that are as one piece with the body.

According to another advantageous characteristic in combination or as an alternative to the other above characteristics, the inner sleeves are sewn to said body by blind stitching, such to allow the positioning of the lips to be guaranteed while keeping the necessary tightness of the device when worn.

According to another advantageous characteristic in combination or as an alternative to the other above characteristics, the device comprises a rigid ring substantially mounted in a central position on the body, such to guarantee a given distance among the inner walls of the device and the catheter, when worn.

According to another advantageous characteristic in combination or as an alternative to the other above characteristics, neoprene is of the type having a thickness ranging from 2 to 5 mm, preferably equal to about 3 mm, such to be thick enough to guarantee the necessary protection, also mechanical protection and a strength enough to be fitted on the arm, while not being too much rigid.

According to another advantageous characteristic that can be provided in combination with one or more of the characteristics described above, at the sealing lips it is possible to provide an annular element tightening them against the limb and having a tightening active condition and a widening inactive condition.

Advantageously the annular element is made as an inflatable and deflatable element, that in the inflated condition generates a predetermined pressure contacting the corresponding sealing lip against the limb which pressure is intended to generate the desired tightness and in the deflated condition it widens releasing the limb and making easier to insert the limb.

The annular element can be fastened inside the sealing lips or outside them.

The fastening can occur by a chemical physical adhesion or by sewing it or said ring can be integrated in the sealing lips.

According to a variant embodiment, the device according to the present invention can be made also as a sleeve that is as a cylinder more narrow at the ends than the central part, for example as it was two joined truncated cones, without providing the folding tabs therein and that is made of smooth neoprene outside and it is lined in the inner side. The device according to such variant can be worn more easily and the tightness is guaranteed by the fact of manually folding towards the inside of the arm two end portions of predetermined length at the two ends of the sleeve. The latter thus act as a seal.

Such solution even if simplified with respect to the those described above, represents a compromise between a higher easiness in wearing action with respect to the previous embodiments where the folding tabs - necessarily made of neoprene - guarantee a perfect tightness and a sufficient watertight seal.

For all the previous described variant embodiments, the term neoprene is the trade name of an elastomer belonging the family of synthetic rubbers based on polychloroprene (polymer form of Chloroprene).

Neoprene (originally called as duprene) was the first residual of synthetic rubber produced on large scale. It belongs to the family of synthetic elastomers (that is synthetic rubbers) and it is like a porous rubber, whose mass is composed of uniformly distributed gas cells. The main characteristics are elasticity, tear and compression resistance, weathering resistance and heat resistance, and moreover it is inert to many chemical agents, oils and solvents.

Further advantageous characteristics are the subject matter of the annexed claims, that are an integral part of the present invention.

### BRIEF DESCRIPTION OF THE DRAWINGS

The invention will be described below with reference to some not limitative examples, provided by way of example and not as a limitation in the annexed drawings. These drawings show different aspects and embodiments of the present invention and, where appropriate, reference numerals showing like structures, components, materials and/or elements in different figures are denoted by like reference numerals.

In the annexed figures:
Figure 1 is a perspective view of a first embodiment of a device according to the invention;
Figure 2 is a phantom view of the device of fig.1 in the worn condition;
Figures 3 and 4 are sections of the device of fig.1 in the non worn and worn conditions respectively;
Figures 5 and 6 are sections of a second embodiment of the device of the invention, in the non worn and worn conditions respectively;
Figure 7 likewise figure 6 is one embodiment where an inflatable and deflatable annular element tightening the sealing lips against the limb is provided inside the sleeve but outside the sealing lips.

### DETAILED DESCRIPTION OF THE INVENTION

While the invention is susceptible of various modifications and alternative constructions, some preferred embodiments are shown in the drawings and will be described in details herein below.

It should be understood, however, that there is no intention to limit the invention to the specific disclosed embodiment but, on the contrary, the invention intends to cover all the modifications, alternative constructions and equivalents that fall within the scope of the invention as defined in the claims.

The use of "for example", "etc.", "or" denotes non-exclusive alternatives without limitation, unless otherwise noted.

The use of "comprises" means "comprises, but not limited to", unless otherwise noted.

Terms as "vertical" and "horizontal", "upper" and "lower" (with no other indications) have to be read with reference to the assembling (or operating) conditions and with reference to the standard terminology in use in common speech, where "vertical" means a direction substantially parallel to that of the vector of the force of gravity "g" and horizontal means a direction perpendicular thereto.

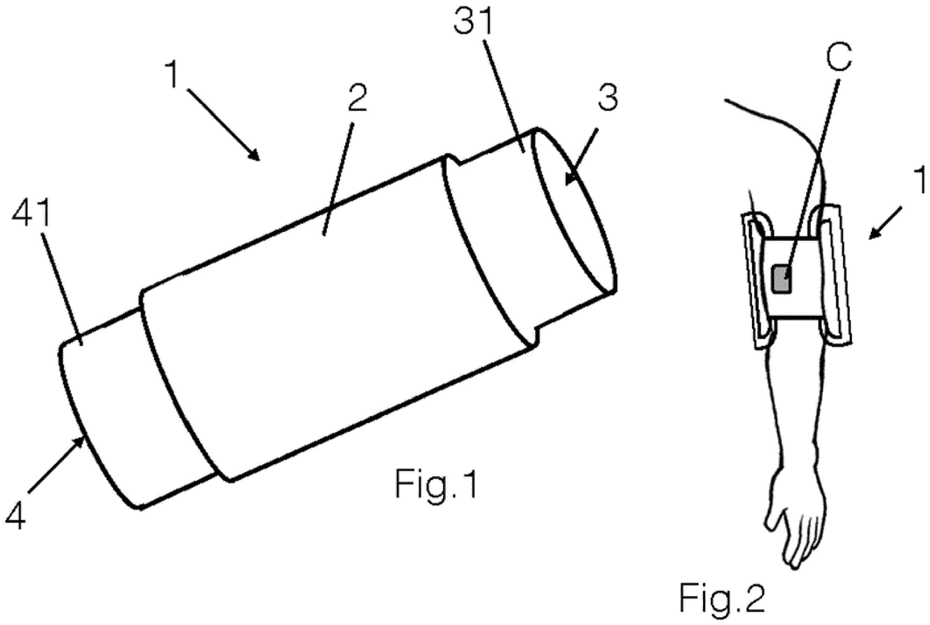

With reference to figs. 1-4 they show with several views a first embodiment of the device of the invention.

The protective device for catheters according to the invention is shown in the first embodiment by the reference numeral 1.

The device 1 comprises a tubular sleeve that in turn comprises a tubular body 2 made of neoprene, comprising at least two opposite open orifices 3, 4, the user arm passing therethrough, such as shown in fig.2.

At the open orifices 3 and 4 there are provided two sealing annular lips 31 and 41; the latter are made of smooth neoprene, such to generate a watertight seal when in contact with the user skin.

A person skilled in the art can select the maximum roughness level of neoprene based on the functionality just described, without for this reason departing from the scope of the present invention; to this end the person skilled in the art can refer for example to neoprene or latex used in waterproof wetsuits in the area of wrists and/or ankles (where waterproof wetsuits have to generate a watertight seal against the user skin).

As it is immediately noted in figs. 1, 3 and 4 each sealing annular lip made of smooth neoprene develops in the area of the body 2 immediately adjacent to the respective orifice 3, 4 and, when the device is not in the worn condition, it is placed on the outer surface of the body 2 particularly in an area of the outer surface immediately adjacent to the respective orifice 3, 4.

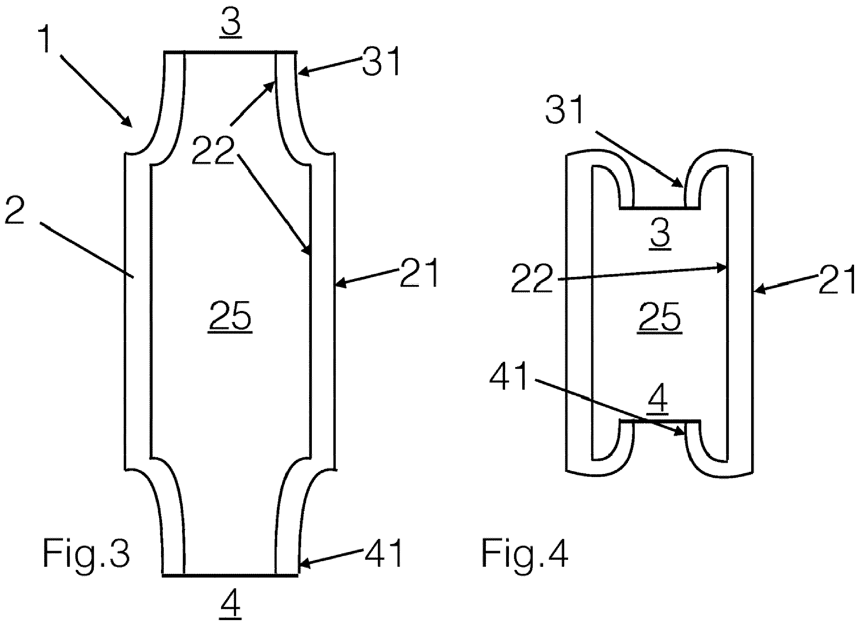

Advantageously the opposite ends of the body 2 provided with the orifices 3, 4, when in use, (such as in fig. 2 and 4) are folded such to bring the sealing lip 31, 41 in contact with the user skin, thus generating the desired tight seal.

The inner surface 22 of the body 2 advantageously is made of lined neoprene, likewise the one used for wetsuits.

The combination of an inner surface 22 made of lined neoprene and of sealing lips 31, 41 made of smooth neoprene placed (in the non worn condition) outside the body 2, allows the step positioning the device 1 on the user arm to have a considerable advantage: the lined neoprene easily runs on the user skin and it does not hinder operations for wearing the device; once the device 1 is placed at the proper height on the arm, its terminal ends provided with sealing lips 31, 41 are therefore folded, such to bring the respective sealing lips 31, 41 in contact with the skin and such to tightly seal the inner cavity 25, where the catheter C is placed.

Moreover another advantage has to be noted: when the device 1 is worn, the catheter portion coming out from the arm is in contact with the inner surface 22 of the body 2, which is lined: this reduces friction and it avoid pains and discomfort for the patient also during the use.

Moreover by the fact that the sealing lips 31, 41 are folded, the inner cavity 25 with the device 1 in the worn condition is slightly wider in comparison with the device 1 being not worn: this avoid a too tight contact between the protruding part of the catheter C and the device 1.

As regards the outer surface 21 of the body 2, remote from the orifices 3 and 4 and from the respective sealing lips 31, 41 in the embodiment of figs.1-4 it is advantageously smooth substantially indistinguishable at the eyes from the sealing lips 31, 41 (that are therefore a portion of the same outer surface 21) ; this allows the device to be cheaper in the manufacturing step.

In other embodiments on the contrary the outer surface 21 of the body 2 remote from the orifices 3 and 4 and from the respective sealing lips 31, 41 is made of neoprene covered by other materials and/or rough neoprene and/or lined neoprene.

In another, more simple, embodiment, not shown, the inner surface is made of smooth neoprene: in this case some of the advantages listed above are not provided (and related to the folding of the ends to bring the sealing lips 31, 41 in contact with the skin), however all the same advantages are provided in comparison to known solutions, especially those related to a more simple operation and to the overall simplicity.

Although in the embodiment shown in figs. 1-4 the end portions of the body 2 are shown with dimensions smaller with respect to the central portion of the body itself, in other embodiments they have the same dimensions.

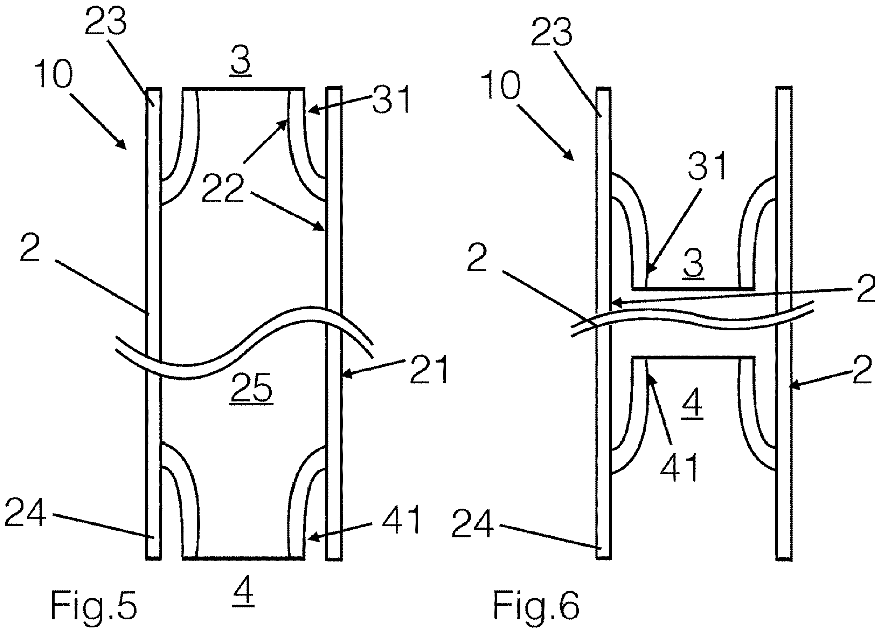

With reference now to a more evolved embodiment, it is shown in figures 5 and 6 and is generally denoted by reference numeral 10.

The parts in common with the base embodiment of the device 1 are denoted by the same reference numerals and have the same functionality described above and therefore no further reference is made thereto.

The difference between the device 10 and the device 1 is the provision of two annular tubular extensions 23 and 24 connected to the body 2 and arranged at the end portions thereof where the orifices 3 and 4 open.

The annular tubular extensions 23 and 24 are substantially positioned such to surround the orifices 3 and 4 and, in the case the overall shape of the device 10 is substantially cylindrical, they are concentric and external to the end portions of the body 2 where the sealing lips 31, 41 are provided.

The annular tubular extensions 23 and 24 can be made in several manners and can be made of different materials, but preferably such extensions 23 and 24 are made of the same material as the body 2.

In some embodiments they are as one piece with the body 2 which is made as a cylinder made of neoprene to which two more internal sleeves are connected for example by sewing them, that carry the sealing lips 31, 41 and that therefore become a part of the body 2, even if not as one piece.

On the contrary in other embodiments the annular tubular extensions 23 and 24 are connected, for example by sewing them, to the body 2 that has the ends provided with the sealing lips 31, 41 as one piece therewith.

Anyway the provision of the annular tubular extensions 23 and 24 developing about the sealing lips 31, 41 helps in making the device 10 more easy to be worn, since the user has two grasp edges (composed of the extensions 23 and 24) at his/her disposal that are free from the sealing lips 31, 41 and therefore are more easy to be handled when wearing the device 10.

Other advantages of the device 10 are substantially similar to that of the device 1 and therefore no further reference is made thereto.

Thus the objects listed above are achieved.

Obviously several variants to what described up to now are possible.

For example the sealing lips 31, 41 can be applied on a body made of lined neoprene, for example by sewing or gluing them.

Still, the body 2 can be provided with a rigid ring (for example made of plastic) intended to guarantee a necessary distance of its inner surface 22 from the catheter C once it is worn; to this end the rigid ring is substantially mounted in a central position on the body 2 and connected to the its shell wall.

The overall length of the device 1, 10 can be manufactured as one desired, for example from 10 to 25 cm in the case, in the worn condition, is has to take the part of the arm from the elbow to the shoulder, while in other versions it goes up to about 30-45 cm, taking the whole arm and covering also possible catheters placed at the elbow or wrist of the user; obviously dimensions will be selected depending on needs and on knowledges of the person skilled in the art, without for this reason departing from the scope of the present invention.

As regards the material, neoprene is preferably of the type having a thickness ranging from 2 to 5 mm, preferably equal to about 3 mm, but it can be selected also with different thicknesses.

As regards connections necessary for making the body 2 and possibly for applying the sleeves that carry the sealing lips or that - in device 10 - are the annular tubular extensions 23 and 24 (depending on cases), such connections can be of the glued type or as an alternative or in combination, can be stitches, preferably of the blind type, such as the ones for wetsuits.

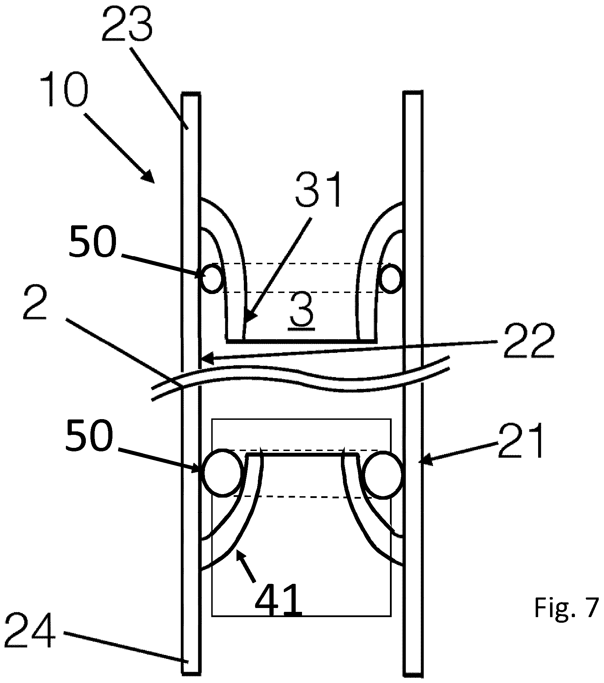

Figure 7 shows a variant that can be provided in combination with all the different embodiments described. The variant is schematically shown and it provides an annular element 50 tightening each sealing lip 31, 41 against the limb.

The annular element can be of the inflatable and deflatable type and the change in diameter causes the corresponding sealing lip to be pressed against the limb.

As it is noted in the figure, in the upper half, the annular element 50 associated with the sealing lip 31 is in the deflated condition, while the one associated to the sealing lip 41 in the lower half of the figure is in the inflated condition, that is expanded condition.

Inflation and deflation can occur by means of a small separable pump and through inflation/deflation nozzles, or the pump can be firmly integrated with the sleeve and fastened therewith.

The schematically shown embodiment has not to be intended as a limitation. The annular tightening element 50 can change its size also by means of other chemical physical phenomena.

Moreover the fastening can occur also in other areas of the sleeve, that is outside the sleeve and also inside the sealing lip.

In a variant the annular element can be integrated with the sealing lips since the latter are made of two walls tightly delimiting an inflation/deflation chamber forming the annular tightening element or that simply houses an annular tightening element.

## Claims

1. Wearable catheter protective device (1, 10), comprising
- a tubular body (2) made of neoprene comprising two opposite open orifices (3, 4)
- two annular sealing lips (31, 41) provided in proximity of such open orifices (3, 4),
said annular sealing lips (31, 41) being made of smooth neoprene, such to generate a water-tight seal when in contact with the skin of a user.

2. Protective device (1, 10) according to the preceding claim, wherein the tubular body (2), in the non-worn condition, has an inner surface (22) and an outer surface (21) and wherein each annular sealing lip (31, 41) is provided on the outer surface (21) of the body (2) at the opposite ends thereof, such that, in the worn condition, said opposite ends are folded to place the sealing lip (31, 41) in contact with the user's skin.

3. Protective device (1, 10) according to claim 2, wherein the inner surface (22) of the body (2) is made of lined neoprene.

4. Protective device (1, 10) according to one or more of the preceding claims, wherein the sealing lips (31, 41) are as one piece with the body (2).

5. Protective device according to one or more of the claims 1 to 4, wherein the sealing lips (31, 41) are applied to the body (2).

6. Protective device (10) according to one or more of the preceding claims, comprising two annular tubular extensions (23, 24) connected to the body (2) and arranged at the end portions thereof provided with the orifices (3, 4).

7. Protective device (10) according to the preceding claim, wherein the annular tubular extensions (23, 24) are as one piece with the body (2), which further comprises two inner sleeves on which said sealing lips (31, 41) are provided.

8. Protective device (10) according to the preceding claim, wherein said inner sleeves are sewn to said body (2) by blind stitching.

9. Protective device (1, 10) according to one or more of the preceding claims, comprising a rigid ring substantially mounted in a central position on the body (2).

10. Protective device (1, 10) according to one or more of the preceding claims, wherein neoprene is of the type having a thickness ranging from 2 to 5 mm, preferably equal to about 3 mm.

11. Device according to one or more of the preceding claims, wherein an annular tightening element (50) brought by said sleeve or by the sealing lip is associated to each sealing lip (31, 41), which annular tightening element has two operating conditions of which an active tightening condition where it presses with a predetermined pressure the corresponding sealing lip against the limb and an inactive condition where said annular tightening element exhibits an increase in the inner diameter that removes the compression of the sealing lip on the limb.
